(19) Europäisches Patentamt
European Patent Office
Office européen des brevets

(11) Publication number: **0 091 071**
**B1**

# (12) EUROPEAN PATENT SPECIFICATION

(45) Date of publication of patent specification: **23.07.86**

(51) Int. Cl.[4]: **C 07 D 451/00** // A61K31/46

(21) Application number: **83103107.5**

(22) Date of filing: **29.03.83**

## (54) Process for the preparation of 5-methyl-10,11-dihydro-5H-dibenzo(a,d)-cyclohepten-5,10-imine and its salts.

(30) Priority: **07.04.82 US 366252**
**03.09.82 US 414739**

(43) Date of publication of application:
**12.10.83 Bulletin 83/41**

(45) Publication of the grant of the patent:
**23.07.86 Bulletin 86/30**

(84) Designated Contracting States:
**CH DE FR GB IT LI NL**

(58) References cited:
**EP-A-0 019 866**
**DE-A-2 840 786**
**US-A-3 879 462**

(73) Proprietor: **MERCK & CO. INC.**
**126, East Lincoln Avenue P.O. Box 2000**
**Rahway New Jersey 07065 (US)**

(72) Inventor: **Bender, Dean R.**
**40 Monterey Drive**
**Hazlet New Jersey 07730 (US)**
Inventor: **Karady, Sandor**
**348 Longview Drive**
**Mountainside New Jersey 07092 (US)**
Inventor: **Rothauser, Theresa**
**606 Goodmans Crossing**
**Clark New Jersey 07066 (US)**

(74) Representative: **Blum, Rudolf Emil Ernst et al**
**c/o E. Blum & Co Patentanwälte Vorderberg 11**
**CH-8044 Zürich (CH)**

**EP 0 091 071 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

BACKGROUND OF THE INVENTION

This invention is concerned with a novel process for 5-methyl-10,11-dihydro-5H-dibenzo-[a,d]cyclohepten-5,10-imine, and its pharmaceutically acceptable salts which is useful as an anxiolytic, antidepressant, anticonvulsant, muscle relaxant, and in the treatment of mixed anxiety-depression, minimal brain dysfunction and extrapyramidal disorders such as Parkinson's disease.

The product of the novel process of this invention, 5-methyl-10,11-dihydro-5H-dibenzo-[a,d]cyclohepten-5,10-imine and its enantiomers are known in the prior art such as *Christy et al., J. Org. Chem.; 44,* 3117-3127 (1979), South African Patent 78/5291 and its West German counterpart Offenlegungschrift 28 40786. The best process known for synthesis of the racemate consists of nine steps from 5H-dibenzo[a,d]cyclohepten-5-one with an overall yield of about 23%. Another process disclosed in EP published Application 0019866 prepares the compound in 5 steps with an overall yield of about 4% of theoretical.

Now, with the present invention, there is provided a novel process for the preparation of 5-methyl-10,11-dihydro-5H-dibenzo-[a,d]cyclohepten-5,10-imine and salts thereof which yields the desired final product in an improved yield.

DETAILED DESCRIPTION OF THE INVENTION

An object of the present invention is a process for the preparation of the racemic compound of structural formula 5

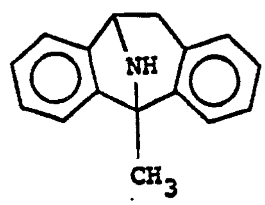

5

and salts thereof which is characterized in that (a) a compound of structural formula 3

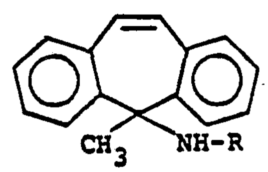

3

wherein

R is either a group of formula —$OR^1$ or a group of formula —$NR^2R^3$ wherein

$R^1$ is hydrogen or an alkyl group with 1—3 carbon atoms

$R^2$ and $R^3$ are independently hydrogen, an alkyl group having 1—3 carbon atoms, an alkanoyl group having 2—3 carbon atoms or a benzenoid aroyl or

$R^2$ and $R^3$ taken together represent tetramethylene or pentamethylene,

is treated in a benzenoid aromatic solvent, dimethylsulfoxide, dimethylformamide or mixtures thereof either with a strong base at a temperature of 0°—190°C or

heated in said solvent without a strong base above about 100°C to produce the compound of structural formula 4:

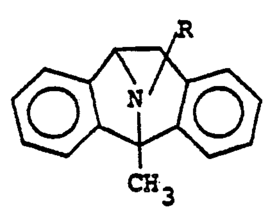

4

and that

(b) the compound of formula 4 is reacted with hydrogen in a solvent in the presence of a noble metal catalyst to yield the compounds of formula 5.

In the starting materials of formula 3 of said process, the radical R is preferably —OH, —$OCH_3$, —$NH_2$ or —$NHCOC_6H_5$ and specifically preferred is a radical R being hydroxy.

The compounds of formula 3 are preferably prepared by reacting a corresponding hydroxy compound and optionally substituted hydroxyl amines or hydrazides.

According to a preferred embodiment of the above stated process, accordingly, the compound of formula 2:

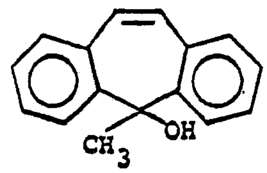

2

is reacted with an amine of formula 6:

$NH_2$—R 6

wherein

R has the same meaning as stated above for the starting material of formula 3 and said reaction is performed in the presence of an intermediate strength organic acid in a chlorinated alkane having 1—3 carbon atoms at −20° to +50°C to yield a substituted amino compound of structural formula 3:

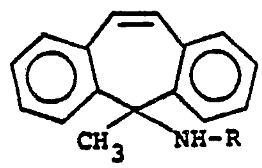

3

which is thereafter submitted to the reaction steps 1 and 2 of the previously described process yielding the desired final product of formula 5.

The carbinol of structural formula 2 of said process can be prepared by submitting a corresponding ketone to a Grignard reaction with a methylmagnesium halide.

According to a further preferred embodiment of the claimed process, accordingly, the ketone of formula 1

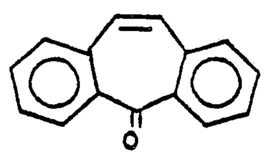

1

is reacted with a methylmagnesium halide in an ethereal solvent at a temperature in the range of −50° to +50°C to yield the carbinol of structural formula 2:

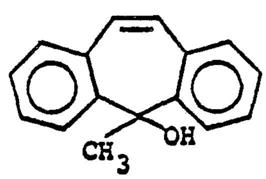

2

which carbinol of formula 2 is thereafter submitted to the four step reaction.

According to said four step process the desired 5-methyl-10,11-dihydro-5H-dibenzo-[a,d]cyclohepten-5,10-imine, can be prepared starting from 5H-dibenzo[a,d]cyclohepten-5-one with an overall yield of about 64%. The above stated processes are illustrated by the following Flow Sheet 1:

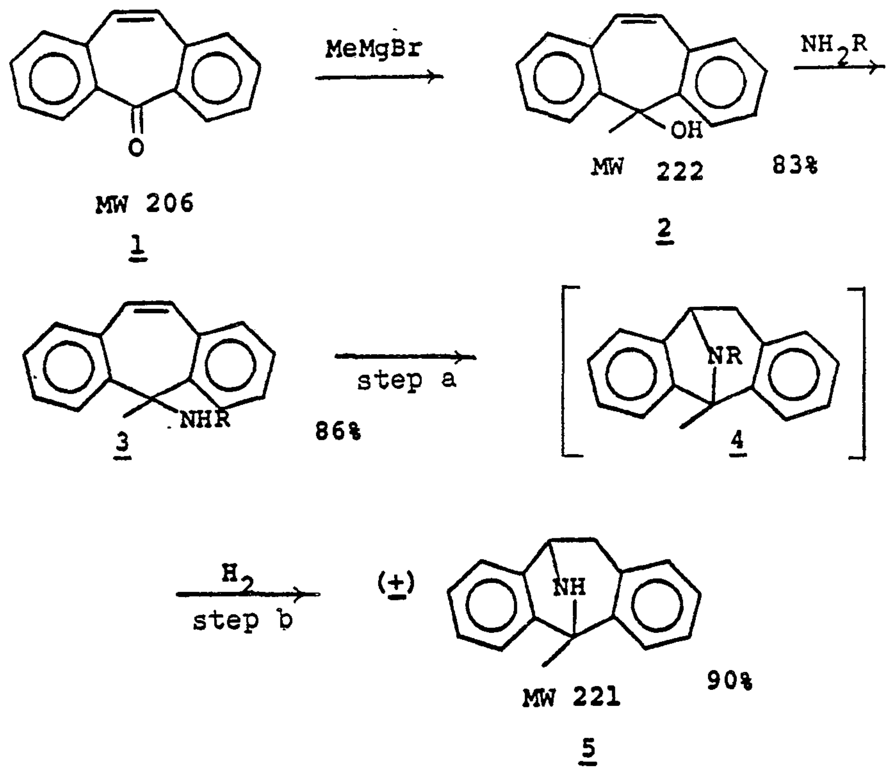


One alternate inventive process for the preparation of the racemic compound of formula 5:

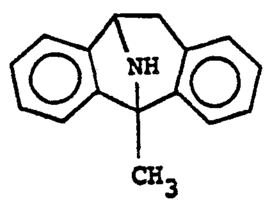

5

or salts thereof, is characterized in that the compound of formula 7:

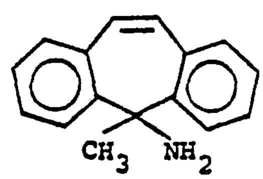

7

is reaction with a strong base in a benzenoid aromatic solvent, dimethylsulfoxide, dimethylformamide or mixtures thereof at a temperature in the range of 0° to 190°C to yield the product of formula 5.

In said process the amino starting material of formula 7 can be prepared by reducing a corresponding azido compound.

According to a further embodiment of the above stated process, accordingly, the compound of formula 8:

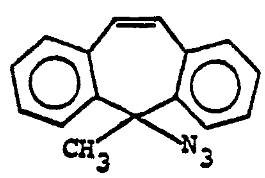

8

is reacted with hydrogen in a solvent in the presence of a noble metal catalyst to produce the compound of structural formula 7:

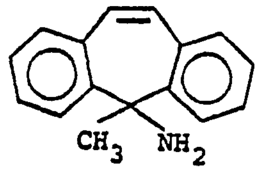

7

and thereafter the compound of formula 7 is submitted to the above stated process to yield the desired final product of formula 5.

The hydrazides of formula 8 are preferably prepared by reacting a corresponding hydroxy compound having the formula 2, with sodium azide. The used hydroxy compound of formula 2 is identical with the hydroxy compound of formula 2 used in the flow sheet 1 to prepare the amino intermediate product of formula 3.

According to a further embodiment of the above stated process, accordingly, the compound of structural formula 2:

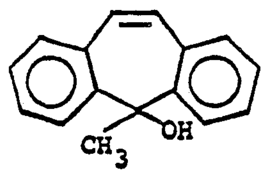

2

is reacted with sodium azide and an organic acid in a chlorinated alkane having 1—3 carbon atoms at a temperature in the range of −20° to +50°C to yield a compound of formula 8:

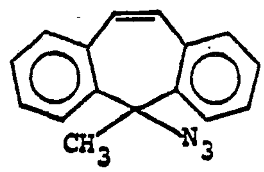

8

and thereafter said compound of formula 8 is submitted to the reactions described above to yield the amino compound of formula 7 and finally the racemic compound of formula 5. As already explained in connection with the flow sheet 1, the carbinol of formula 2 can be prepared by submitting a corresponding ketone of formula 1 to a Grignard reaction with a methylmagnesium halide.

According to a further preferred embodiment of the above mentioned process, accordingly, the compound of structural formula 1:

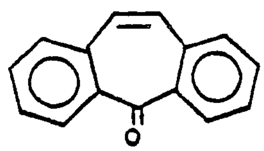

1

is reacted with a methylmagnesium halide in an ethereal solvent at a temperature in the range of −50° to +50°C to produce the carbinol of formula 2:

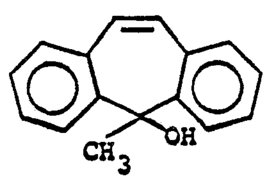

2

and thereafter said carbinol of formula 2 is submitted to the further reaction steps to yield the azide of formula 8, the amine of formula 7 and finally the racemic compound of structural formula 5.

Said alternate procedure is illustrated by the following flow sheet 2:

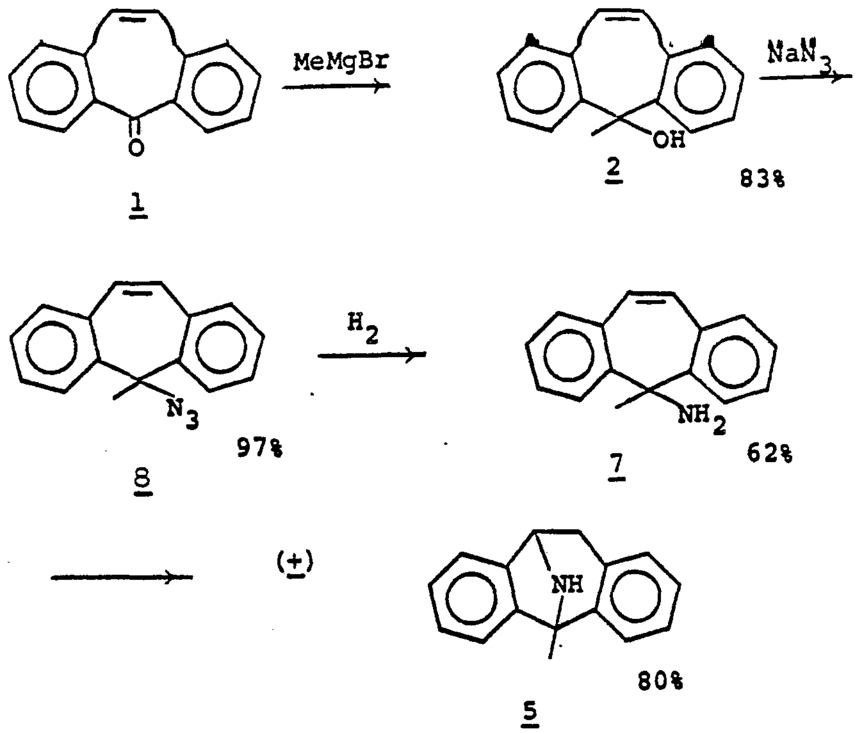


REAGENTS AND CONDITIONS

Performance of the Grignard reaction with methylmagnesium halide

The ketone of formula I is reacted with the methylmagnesium halide, preferably the bromide, in an ethereal solvent such as diethyl ether, tetrahydrofuran, 1,2-dimethoxyethane, or the like. The reaction is conducted at −50°C to about +50°C, preferably at about 15—25°C until the reaction is substantially complete; about 1—2 hours at 15—20°C. Aging the reaction for much longer periods such as up to about 24 hours is not deleterious.

Formulation of the substituted amino compound 3 according to flow sheet 1

A mixture of the amine of formula 6 and the carbinol of formula 2 is reacted in the presence of an immediate strength organic acid such as dichloroacetic acid, trichloroacetic acid, difluoroacetic acid, or the like, or with a mixture thereof. The reaction is performed in a chlorinated alkane having 1—3 carbon atoms, for example methylene chloride, 1,2-dichloroethane, chloroform or the like, at about −20 to +50°C, preferably at about 15—25°C until the reaction is substantially complete. The reaction proceeds rapidly being complete in about an hour.

Formation of the azide of formula 8 of flow sheet 2

The carbinol of formula 2 is reacted with sodium azide in an organic acid, preferably a stronger organic acid such as trifluoroacetic acid. The reaction is performed in a chlorinated alkane having 1—3 carbon atoms, for example methylene chloride, 1,2-dichloroethane or chloroform at about −20 to +50°C, preferably at about 15—25°C until the reaction is substantially complete. The reaction proceeds rapidly being complete in about an hour.

Performance of the cyclization of the substituted amino compound of formula 3 of flow sheet 1

The substituted amino compound 3 is treated in a solvent or mixture of solvents with a strong base such as an alkali metal hydride, amide or alkoxide, especially potassium *tert*-butoxide. Preferably the amino compound 3 is added to a solvent system composed of a benzenoid aromatic solvent such as toluene, benzene or the like, dimethylformamide, dimethyl-sulfoxide or mixtures thereof which contains said strong base. The reaction is performed at about 0° to about 190°C, preferably about 50 to 125°C until the reaction is substantially complete. Times of up to 20 hours or longer may be employed.

The cyclization of the substituted amine of formula 3, however, can be also performed by treating it without a strong base in the above mentioned solvent or solvent system above about 100°C. The heating is performed until the ring closure is substantially complete, in about 10 to 20 hours. Refluxing toluene is preferred. The cyclization is preferably conducted in an inert atmosphere, such as under nitrogen.

Hydrogenolysis of the N—R group of the compounds of formula 4 in flow sheet 1

A compound of formula 4 is reacted with hydrogen in a solvent in the presence of a noble metal catalyst such as palladium on carbon, platinum on carbon, platinum oxide, Raney nickel or the like,

preferably 5% palladium on carbon to yield a compound of formula 5. The catalytic hydrogenation is performed in a solvent such as acetic acid or ethanol or mixtures thereof, with or without a strong mineral acid such as hydrochloric acid, sulfuric acid, phosphoric acid or the like. The hydrogenation is preferably performed at about 20—100°C, e.g. about 60°C. The hydrogenation is continued until the rate of hydrogen uptake substantially slows and the amount consumed is approximately theoretical.

Hydrogenation of the azide of formula 8 in flow sheet 2

The hydrogenation of the compound of formula 8 is performed in a solvent in the presence of a noble metal catalyst and said catalytic hydrogenation is preferably performed in the same way as outlined above for the catalytic hydrogenation of the compound of formula 4 in order to produce the final product of formula 5 in flow sheet 1.

Cyclization of the amino compound 7 of flow sheet 2

Said cyclization is performed by reacting a compound of formula 7 with a strong base in a benzenoid aromatic solvent, dimethylsulfoxide, dimethylformamide or mixtures thereof at a temperature in the range of 0° to 190°C to yield the product of formula 5. Said reaction can be performed in a similar way as the corresponding cyclization reaction of the amine of formula 3 in flow sheet 1. Accordingly, as strong base there can be used an alkali metal hydride, amide or alkoxide, especially potassium *tert*-butoxide. The benzenoid aromatic solvent can e.g. be toluene or benzene and the reaction has to be performed at a temperature of about 0° to about 190°C, preferably about 50 to 125°C until the reaction is substantially complete. Times of up to 20 hours or longer may be employed.

The product of the novel process of this invention is a racemic mixture, resolvable into its enantiomers by standard techniques such as the formation of diastereomeric salts with an optically active acid, such as (−)-di-p-toluoyl-d-tartaric acid and/or (+)-di-p-toluoyl-1-tartaric acid followed by fractional crystallization and regeneration of the free base.

The product of the novel process of this invention and its dextrorotatory enantiomer are capable of producing anxiety relief without causing excessive sedation or sleep at a dosage level of from about 0.01 to about 20 mg. per kilogram of body weight preferably about 0.05—2 mg/kg of body weight on a regimen of 1—4 times a day. In addition, the product of the present invention and the dextrorotatory enantiomer are useful as antidepressants, muscle relaxants, anticonvulsants and in the treatment of mixed anxiety-depression, minimal brain dysfunction and extrapyramidal disorders, such as Parkinson's disease, when indicated, at comparable dosage levels. It is understood that the exact treatment level will depend upon the case history of the animal or human individual being treated and in the last analysis the precise treatment level falling within the above guidelines is left to the discretion of the therapist.

Example 1

Step A:
*5-Methyl-5H-dibenzo[a,d]cyclohepten-5-ol (2)*

Materials:

| | | |
|---|---|---|
| 5H-Dibenzo[a,d]cyclohepten-5-one (*1*), "trienone" | 875.5g | 4.25mol |
| Methylmagnesium bromide 2.28*M* in diethylether | 7.11 g | 5.96 mol |
| Ammonium chloride 5.0*M* | 954 g | 17.84 mol |
| Tetrahydrofuran (sieve dried) | 3.79 l. | |
| Hexane | 12 1. | |
| Ethyl acetate | 5.65 l. | |

To 1.42 l. of dry THF was added with cooling ($T_i$ 5—10°C), 5.96 mol (2.28*M* in diethylether, 711 g) of methylmagnesium bromide. A solution of 875.5 g (4.25 mol) of "trienone" (*1*) in 2.37 l. dry THF was then added over a 1.75 hour period to the above mixture, maintaining the temperature between 15—20°C. The reaction mixture was brought to room temperature and was then stirred overnight.

The reaction mixture was cooled and an aqueous solution of 5.0*M* ammonium chloride (954 g, 17.85 mol) was slowly added keeping the temperature at less than 30°C.

The organic layer was separated and the aqueous layer, to which NaCl was added, was washed with 3 × 1.8 l. ethyl acetate.

The organic layers were combined, dried over $Na_2SO_4$, filtered and concentrated under reduced pressure to approximately 1.75 l. (slurry). To this was added 0.25 l. ethyl acetate, which was warmed redissolving all solids. The solution was then cooled to less than 50°C and 10 l. of hexane were added in portions, forming a white fluffy precipitate. This was further chilled (0 to 5°C) for approximately 1/2 hour. The precipitate was filtered and washed with 2 × 1 l. cold hexane (0 to 5°C), and air dried at 50—55°C, to give 783 g (83%) of *2*, as a white crystalline solid, m.p. 113.5—115°C.

Step B:
*5-Methyl-5-hydroxamino-5H-dibenzo[a,d]cycloheptene (3)*

| Materials: | | |
|---|---|---|
| 5-Methyl-5H-dibenzo[a,d]-cyclohepten-5-ol (2) | 60.0 g | 0.270 mol |
| Dichloroacetic acid | 0.138 l | 1.617 mol |
| Hydroxylamine hydrochloride | 75.11 g | 1.081 mol |
| Sodium acetate | 88.61 g | 1.081 mol |
| Methylene chloride | 2.5 l. | |
| Hexane | 0.8 l. | |
| Concentrated ammonium hydroxide | 0.3 l. | |

To a mixture of 0.138 l. $CH_2Cl_2$ and 0.138 l. (1.617 mol) of dichloroacetic acid was added with cooling ($T_i$ 20°C) 88.61 g (1.081 mol) of sodium acetate. After dissolving the sodium acetate, 75.11 g (1.081 mol) of hydroxylamine hydrochloride was added. The slurry was stirred for 1.5 hours at room temperature and an additional 1.362 l. of $CH_2Cl_2$ wa added over approximately 10 minutes.

The reaction mixture was then stirred over an additional 1.5 hour period. A solution of 60.0 g (0.270 mol) of the alcohol *2* in 0.600 l. was added to the above reaction mixture over 10 minutes. The reaction was complete after 0.5 hours.

The reaction mixture was quenched with 0.9 l. ice water and 0.3 l. concentrated $NH_4OH$ (pH~8).

After stirring, the separated aqueous layer was washed with 1 × 0.4 l. $CH_2Cl_2$, the organic layers combined, washed with 1 × 0.1 l. brine and dried over $Na_2SO_4$. The reaction mixture was filtered and concentrated under reduced pressure to approximately 0.10 l. To this was added in portions with stirring 1.1 l. hexane, forming a white precipitate. This was chilled (0 to 5°C) for 1 hour, filtered, washed with 0.15 l. cold hexane (0 to 5°C) and air dried at ambient temperature to give 54.8 g (86%) of *3*, a white crystalline solid, which was stored at less than 0°C, m.p. 130—133°C.

Employing the procedure substantially as described in Example 1, Step B, but substituting for the hydroxylamine hydrochloride used therein, equimolecular amounts of amines of formula $NH_2R$ identified in Table I, there were produced the 5-methyl-5-(R-amino)-5H-dibenzo[a,d]cycloheptenes also described in Table I, in accordance with the following reaction scheme:

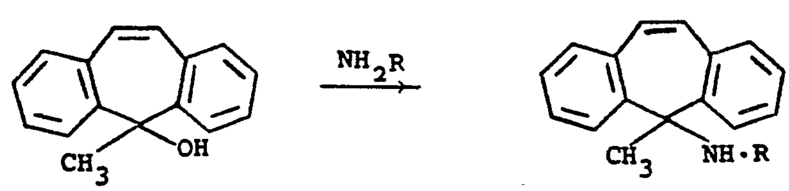

TABLE I

| R | % Yield | Product m.p. (°C) |
|---|---|---|
| $—NH_2$ | 100 | oil(1) |
| $—OCH_3$ | 82 | 118.5—126 |
| $—NHCOC_6H_5$ | 93 | oil(2) |

(1) NMR ($CDCl_3$)δ: 2.05 (s, 3H, $CH_3$), 3.0 (s, 3H, $NHNH_2$), 6.95 (s, 2H, —CH=), 7—7.6 (m, 8H, aromatic).
(2) NMR ($CDCl_3$)δ: 2.15 (s, 3H, $CH_3$), 7.1 (s, 2H, HC=), 7—7.5 (m, 8H, aromatic).

Step C:
*12-Hydroxy-5-methyl-10,11-dihydro-5H-dibenzo[a,d]cyclo-hepten-5,10-imine. (4)*

| Materials | | |
|---|---|---|
| 5-Methyl-5-hydroxamino-5H-dibenzo[a,d]cycloheptene (*3*) | 10.00 g | 0.0422 mol |
| Potassium *tert*-butoxide | 4.76 g | 0.0422 mol |
| 10% Dimethyl sulfoxide/toluene (sieve, dried, v/v) | 100 ml | |
| Toluene | 25 ml | |
| 1:1 (v/v) 1.2*M* HCl/glacial acetic acid | 100 ml | |
| Darko KB charcoal | 1.0 g | |

A mixture of 50 ml of 10% DMSO/toluene (sieve dried, v/v) and 4.76 g (0.0422 mol) of potassium *tert*-butoxide was heated to 55°C and a solution of 10.00 g (0.0422 mol) of hydroxylamine *3* in 50 ml 10% DMSO/toluene was added quickly and rinsed in with 5 ml toluene.

Three minutes after the addition was complete the reaction mixture was removed from the heat and 50 ml of water added. The reaction mixture was allowed to stir until it reached ambient temperature.

The aqueous layer was separated and backwashed with 20 ml toluene to which 5 ml brine was added. The organic layer was washed with 3 × 50 ml water and 1 × 50 ml brine. Each successive aqueous and final brine layer was also back-washed with the 20 ml of toluene. An additional 5 ml of brine was added to the aqueous layers.

The two organic layers were combined, allowed to settle (any additional brine solution remaining was separated) and washed with a 1:1 solution of 1.2*M* HCl (v/v)/glacial acetic acid, 3 × 33.3 ml. The acid layers were combined 1.0 g decolorizing charcoal was added and the mixture stirred 0.5 hours at ambient temperature. This mixture after filtration, was used in the subsequent hydrogenolysis.

Crystalline ring closed hydroxylamine *5* can be isolated at this point by basification of the concentrated acid layer ($NH_4OH$), extraction ($CH_2Cl_2$) and crystallization using a minimum amount of hot $CH_2Cl_2$ and hexane (1:8 ratio respectively) to give 12-hydroxy-5-methyl-5H-10,11-dihydro[a,d]cyclohepten-5,10-imine (*5*) existing as two isomers with combined m.p. 138—141°C.

Employing the procedure substantially as described in Example 1, Step C, but substituting for the 5-methyl-5-hydroxamino-5H-dibenzo[a,d]cycloheptene used therein, equimolecular amounts of the 5-methyl-5-(R-amino)-5H-dibenzo[a,d]cycloheptenes described in Table II, there were produced the 12-R-5-methyl-10,11-dihydro-5H-dibenzo[a,d]cyclohepten-5,10-imines also described in Table II, in accordance with the following reaction shceme:

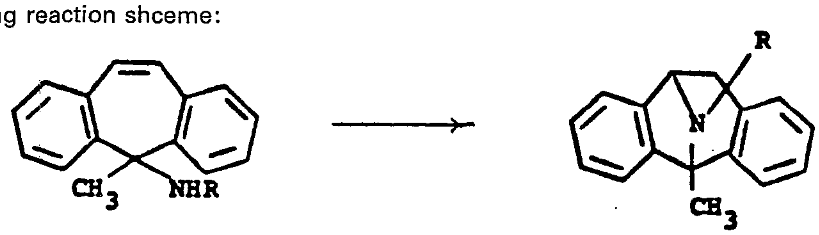

TABLE II

| R | % Yield | Product m.p. (°C) |
|---|---|---|
| $-NH_2$ | 100 | oil(1) |
| $-OCH_3$ | 60 | oil(2) |
| $-NHCOC_6H_5$ | 84 | 216—218 |

(1) NMR ($CDCl_3$)δ: 1.7 (s, 3H, $CH_3$), 2.55 (d, 1H, J=17Hz, —$\underline{CH_2}$—CH—), 3.45 (d of d, 1H, J=6 and 17Hz, —$\underline{CH_2}$—CH), 4.35 (d, 1H, J=6Hz, —$\underline{CH}$—$CH_2$—).
(2) NMR ($CDCl_3$)δ: 1.85 and 1.90 (s, 3H, $CH_3$), 2.7 (d, 1H, J=18Hz, $\underline{CH_2}$—CH), 3.3 (m, partially visible $\underline{CH_2}$—CH), 3.55 (s, 3H, $OCH_3$), 4.6 (d, 1H, J=6Hz, $\underline{CH}$—$CH_2$).

Alternate Step C:
*12-Hydroxy-5-methyl-10,11-dihydro-5H-dibenzo[a,d]cyclohepten-5,10-imine. (4)*

| Materials | | |
|---|---|---|
| 5-Methyl-5-hydroxamino-5H-dibenzo[a,d]cycloheptene (3) | 2.37 g | 0.01 mol |
| Toluene | 200 ml | |

The mixture of hydroxamino compound and toluene was refluxed 12 hours. The mixture was concentrated to 90 ml *in vacuo,* and extracted with 3 × 50 ml of 2N HCl. The extract was adjusted to pH 10 with $NH_4OH$ and extracted with 90 ml $CH_2Cl_2$. The extract was dried over $MgSO_4$, filtered and concentrated to dryness to give the subject compound in approximately 60% yield.

Step D:
*(±)-5-Methyl-10,11-dihydro-5H-dibenzo[a,d]cyclohepten-5,10-imine*

| Materials: | | |
|---|---|---|
| 12-Hydroxy-5-methyl-10,11-dihydro-5H-dibenzo[a,d]cyclohepten-5,10-imine as a solution in 100 ml of 1:1 (v/v) 1.2*M* HCl/glacial acetic acid). | 10.00 g | 0.0422 mol |
| 1:1 (v/v) 1.2*M* HCl/glacial acetic acid | 50 ml | |
| Sodium acetate | 5.4 g | 0.061 mol |
| 5% Palladium-on-carbon (Engelhard) | 1.0 g | |
| Concentrated ammonium hydroxide | 55 ml | |
| Methylene chloride | 150 ml | |

The mixture of 10.00 g (0.0422 mol; based on 100% yield over the previous step) of the ring closed hydroxylamine *4* and 1.0 g of decolorizing charcoal in 100 ml of 1:1 (v/v) 1.2*M* HCl/glacial acetic acid was filtered through diatomaceous earth into a 250 ml Parr bottle. The cake was rinsed with approximately 15—25 ml of the HCl/glacial acetic acid mixture until the total volume of the reaction solution was 120 ml.

To this solution was added with warming 5.4 g (0.061 mol) of sodium acetate and 1.0 g of 5% palladium-on-carbon. The mixture was shaken under pressure at 60°C on the Parr apparatus until the rate of hydrogen uptake clearly slowed.

After approximately 3.25 hours the reaction mixture was degassed with $N_2$, filtered through diatomaceous earth and rinsed with 25 ml of 1:1 (v/v) 1.2*M* HCl/glacial acetic acid. The reaction was concentrated under reduced pressure to one-half its original volume, 100 ml of ice added, the reaction mixture made basic (pH=8) with concentrated ammonium hydroxide (55 ml) and the basic solution extracted 3 × 50 ml with $CH_2Cl_2$. The organic layers were combined, washed with 1 × 50 ml brine, dried over $Na_2SO_4$, filtered and the solvent concentrated under reduced pressure to give 8.4 g (90%) of *5*, a yellow oil which crystallized upon standing, m.p. 77—85°C.

Employing the procedure substantially as described in Example 1, Step D, but substituting for the 12-hydroxy-5-methyl-10,11-dihydro-5H-dibenzo[a,d]cyclohepten-5,10-imine used therein, equimolecular amounts of the 12-R-5-methyl-10,11-dihydro-5H-dibenzo[a,d]cyclohepten-5,10-imines wherein R is —$NH_2$, —$OCH_3$ and —$NHCOC_6H_5$ there is produced 5-methyl-10,11-dihydro-5H-dibenzo-[a,d]cyclohepten-5,10-imine identical with previously prepared material in substantially similar yields in accordance with the following reaction scheme:

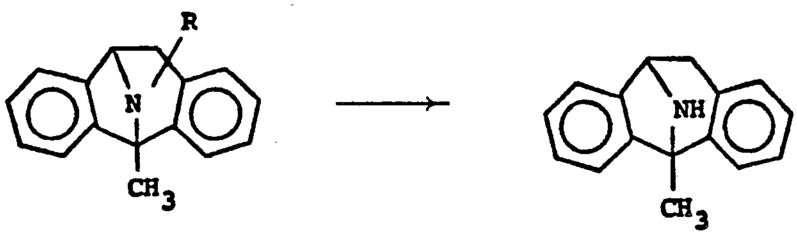

Employing the procedure substantially as described in Example 1, Steps A through D but substituting for the hydroxylamine used in Step B thereof, equimolecular amounts of amines of the formula $NH_2R$, there are produced in sequence the intermediates:

5-methyl-5-(R-amino)-5H-dibenzo[a,d]cycloheptene (*3*);

12-R-5-methyl-10,11-dihydro-5H-dibenzo[a,d]cyclohepten-5,10-imine (*4*) and finally

5-methyl-10,11-dihydro-5H-dibenzo[a,d]cyclohepten-5,10-imine (*5*)

wherein R is:

—$OC_3H_7$-n

—NH—$C_2H_5$

$CH_3$
/
—N
\
$CH_3$

—NH—$COC_2H_5$

—NH—$COC_6H_4CH_3$

Example 2

Step A:

*5-Methyl-5H-dibenzo[a,d]cyclohepten-5-ol (2)*

See Example 1, Step A.

Step B:

*5-Methyl-5-azido-5H-dibenzo[a,d]cycloheptene (6)*

| | | |
|---|---|---|
| Carbinol (1) | 10.0 g | $4.5 \times 10^{-2}$mol |
| $NaN_3$ | 11.6 g | $1.78 \times 10^{-1}$mol |
| $CF_3CO_2H$ | 18.0 mls | $2.11 \times 10^{-1}$mol |

In a 3-necked flask was placed 11.6 g ($1.78 \times 10^{-1}$mol) $NaN_3$ in 0.25 l of $CH_2Cl_2$ under $N_2$. To this was added with cooling (0°C) 18.0 ml ($2.11 \times 10^{-1}$mol) $CF_3CO_2H$ over 5 minutes. The reaction was stirred for 10 minutes at room temperature and 10.0 g ($4.5 \times 10^{-2}$mol) of carbinol in 0.1 l of $CH_2Cl_2$ was added dropwise (15 minutes) at ambient temperature. After 40 minutes, 0.100 l of ice water and 25 ml of concentrated $NH_4OH$ was added (pH>8) and the reaction was stirred for 10 minutes. The organic layer was separated and the aqueous layer extracted with 1 × 30 ml of $CH_2Cl_2$. The organic layers were combined, washed with 1 × 100 ml of brine, dried with $MgSO_4$ and the solvent was removed under reduced pressure to give 11.0 g crystalline product, *6*, (97%).

m.p. 65.5°C—67°C

$^1$HNMR ($CDCl_3$)δ 1.90 (s, 3H, $CH_3$), 6.95 (s, 2H, HC=CH) and 7.00—7.80 (m, 8H, Ar)

IR ($CDCl_3$) 2125 $cm^{-1}$($N_3$).

Step C:
*5-Methyl-5-Amino-5H-dibenzo[a,d]cycloheptane, (7)*

| | | |
|---|---|---|
| Azide 6 | 7.0 g | $2.83 \times 10^{-2}$mol |
| $PtO_2$ | 1.4 g | |
| Absolute Ethanol | 210 ml. | |

7.0 g ($2.83 \times 10^{-2}$mol) of *6* dissolved in 0.21 l of absolute ethanol was charged with 1.4 g $PtO_2$ and placed on a Parr shaker overnight under $H_2$ at 5 psi and ambient temperature. The mixture was filtered through Super-Cel, and the filtrate stripped of solvent. The resulting oil was taken up in 50 ml of $CH_2Cl_2$ and extracted with 3 × 25 ml of 10% HCl. The aqueous layers were combined, basified with concentrated $NH_4OH$, extracted with 3 × 25 ml of $CH_2Cl_2$. The organic layers were combined, dried with $MgSO_4$ and the solvent removed to give 3.85 g of the desired amine *7* in a 62% yield.

$^1$HNMR ($CDCl_3$) 1.77 (s, 3H, $CH_3$), 2.10 (s, 2H, $NH_2$ exchanged by $D_2O$), 7.00 (s, 2H, HC=CH) and 7.05—7.75 (m, 8H, Ar).

IR (neat) 3300—3400 (broad $NH_2$), 1580, 1430 and 1475 $cm^{-1}$.

Step D:
*(±)-5-Methyl-10,11-dihydro-5H-dibenzo[a,d]cyclohepten-5,10-imine (5)*

| | | |
|---|---|---|
| Amine (*7*) | 0.5 g | $2.26 \times 10^{-3}$mol |
| KO*t*Bu | 0.26 g | $2.26 \times 10^{-3}$mol |
| DMSO | 5.0 ml | |

0.26 g ($2.26 \times 10^{-3}$mol) Ko*t*-Bu was placed in 2.5 ml of sieve dried DMSO under $N_2$ and warmed to 75°C. To this was added dropwise 0.5 g ($2.26 \times 10^{-3}$mol) of amine *7* dissolved in 2.5 ml of DMSO. The reaction was heated at 125°C for approximately 18 hours. The reaction was cooled to ambient temperature and 10 ml of $H_2O$, and 10 ml of ethyl acetate added and the reaction mixture was stirred open to the air. The aqueous layer was separated and back washed with 5 ml of ethyl acetate. The original ethyl acetate layer was washed with 2 × 10 ml of $H_2O$. The ethyl acetate back-wash was then extracted with these two 10 ml $H_2O$ washes. Both organic layers were combined and washed with 1 × 10 ml of brine, dried over $MgSO_4$ and stripped of solvent to give 0.4 g of *5* in 80% yield.

OPTICAL RESOLUTION
*Preparation of (+)-5-Methyl-10,11-dihydro-5H-dibenzo[a,d]cyclohepten-5,10-imine.di-*p*-toluoyl-1-tartaric acid.acetone.*

| Materials: | | |
|---|---|---|
| (±)-5-Methyl-10,11-dihydro-5H-dibenzo[a,d]cyclohepten-5,10-imine (*5*) | 284.4 g | 1.29 mol |
| Di-*p*-toluoyl-*1*-tartaric acid-monohydrate | 720 g | 1.78 mol |
| Acetone | 3.25 l. | |
| Methylene chloride | 6.0 l. | |
| Concentrated ammonium hydroxide | 1.5 l. | |

The cyclic amine *5* (284.4 g, 1.29 mol) was dissolved in 402 ml of acetone and filtered through a medium sintered glass funnel into a 3—1. 3-necked flask fitted with a mechanical stirrer and thermometer. An additional 63 ml of acetone was used as a rinse.

A solution of 519 g (1.28 mol) of the resolving acid in approximately 600 ml of warm acetone was similarly filtered into a separate flask with an additional 60 ml used for rinsing. The amine solution was warmed to 40°C and the resolving acid solution added rapidly with stirring ($T_i$ approximately 40°C). The solution was seeded and the reaction mixture brought to ambient temperature with stirring overnight.

The mixture was filtered and the white, finely crystalline product washed with 4 × 200 ml cold acetone

(10°C) and air dried at 55°C to give 331 g (0.497 mol; 39%) of diastereomeric salt $[\alpha]_D^{25}$ = +127.7° (C=1, abs. ethanol).

A solution of 330 g of the enriched salt in 3.0 l. of methylene chloride was added to 6.0 l. of 1:3 concentrated ammonium hydroxide/$H_2O$ (v/v) and stirred. The $CH_2Cl_2$ layer was separated and the basic layer extracted with 2 × 1.5 l. of $CH_2Cl_2$. The organic layers were combined, dried over $MgSO_4$, filtered and concentrated to a viscous oil (119.6 g).

The viscous oil was dissolved in 254 ml of acetone and filtered into a 3 l. 3-neck flask as previously described, using 208 ml of acetone as a rinse. A solution of 200.7 g (0.497 mol) of resolving acid dissolved in 254 ml of acetone was filtered as described previously, using 208 ml acetone as a rinse and added to the amine solution at 42—43°C. The reaction mixture was seeded and brought to ambient temperature with stirring overnight. The white precipitate was filtered, washed with 4 × 100 ml of cold acetone (10°C) and air dried at 50 to 60°C to give 294.2 g (89%) of crystalline salt m.p. 137—138°C (dec); $[\alpha]_D^{25}$ = +132.2° (C=1, abs. ethanol).

*Preparation of (+)-5-Methyl-10,11-dihydro-5H-dibenzo[a,d]cyclohepten-5,10-imine.hydrogen maleate*

| Materials: | | |
|---|---|---|
| (+)-5-Methyl-10,11-dihydro-5H-dibenzo-[a,d]cyclohepten-5,10-imine.di-*p*-toluoyl-*l*-tartaric acid.acetone | 293 g | 0.44 mol |
| Methylene chloride | 5.4 l. | |
| Concentrated ammonium hydroxide | 1.3 l. | |
| Maleic acid (Aldrich) | 51.07 g | 0.44 mol |
| Absolute ethanol | 1.05 l. | |

The amine salt, 293 g (0.44 mol) was dissolved in 2.7 l. $CH_2Cl_2$ to which 5.2 l. of 1:3 concentrated ammonium hydroxide/$H_2O$ was added. The basic layer was separated, washed with 2 × 1.35 l. $CH_2Cl_2$, the organic layers combined, dried over $MgSO_4$, filtered and concentrated under reduced pressure to a viscous oil (105 g).

The viscous oil was dissolved in 282 ml of absolute ethanol and filtered through a medium sintered-glass funnel into a 2—1. 3-necked flask fitted with a mechanical stirrer and thermometer, using an additional 145 ml of absolute ethanol as a rinse.

A solution of 51.07 g (0.44 mol) of maleic acid dissolved in 14l ml of absolute ethanol was filtered as previously described using 78 ml of absolute ethanol as a rinse and added rapidly with stirring to the warmed amine solution (44°C).

The reaction mixture was stirred for 10 minutes ($T_i$ 47°C), seeded and slowly stirred overnight at room temperature. The white precipitate was filtered, washed with 4 × 100 ml cold absolute ethanol (5°C) and dried under vacuum at 50—60°C to give 129.1 g (87%) of crystalline (+)-5-methyl-10,11-dihydro-5H-dibenzo[a,d]cyclohepten-5,10-imine.hydrogen maleate m.p. 205.5—206., 5°C (d) with an observed rotation of $[\alpha]_D^{25}$ = +114.7 (C=1, methanol).

## Claims

1. Process for the preparation of the racemic compound of structural formula 5:

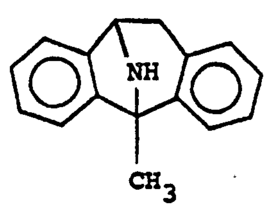

5

and salts thereof characterized in that

a) a compound of structural formula 3

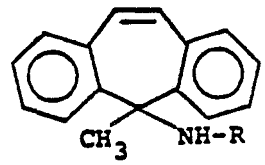

3

wherein

R is either a group of formula —$OR^1$ or a group of formula —$NR^2R^3$ wherein

$R^1$ is hydrogen or an alkyl group with 1—3 carbon atoms

$R^2$ and $R^3$ are independently hydrogen, an alkyl group having 1—3 carbon atoms, an alkanoyl group having 2—3 carbon atoms or a benzenoid aroyl or

$R^2$ and $R^3$ taken together represent tetramethylene or pentamethylene,

is treated in a benzenoid aromatic solvent, dimethylsulfoxide, dimethylformamide or mixtures thereof either with a strong base at a temperature of 0°—190°C or heated in said solvent without a strong base above about 100°C to produce the compound of structural formula 4:

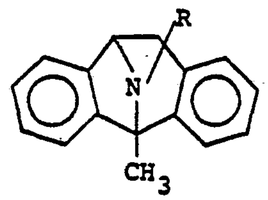

4

and that

b) the compound of formula 4 is reacted with hydrogen in a solvent in the presence of a noble metal catalyst to yield the compounds of formula 5.

2. Process according to claim 1 characterized in that in the compound of formula 3 R is —OH, —$OCH_3$, —$NH_2$ or —$NHCOC_6H_5$.

3. Process according to claim 1 or 2 characterized in that in the compound of formula 3 R is hydroxy.

4. Process according to one of the patent claims 1—3, characterized in that the compound of formula 2:

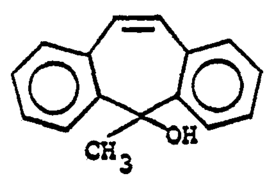

2

is reacted with an amine of formula 6:

$$NH_2—R \qquad 6$$

wherein

R is either a group of formula —$OR^1$ or a group of formula —$NR^2R^3$ wherein

$R^1$ is hydrogen or an alkyl group with 1—3 carbon atoms

$R^2$ and $R^3$ are independently hydrogen, an alkyl group having 1—3 carbon atoms, an alkanoyl group having 2—3 carbon atoms or a benzenoid aroyl or

$R^2$ and $R^3$ taken together represent tetramethylene or pentamethylene, and wherein the reaction is performed in the presence of an intermediate strength organic acid in a chlorinated alkane having 1—3 carbon atoms at −20° to +50°C to yield a substituted amino compound of structural formula 3:

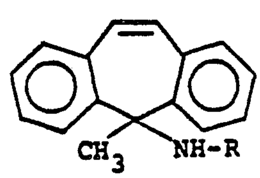

3

which is thereafter submitted to the reaction steps 1 and 2 of claim 1 yielding the final product of formula 5.

5. Process according to patent claim 4 characterized in that the ketone of formula 1

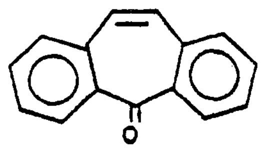

1

is reacted with a methylmagnesium halide in an ethereal solvent at a temperature in the range of −50° to +50°C to yield the carbinol of structural formula 2:

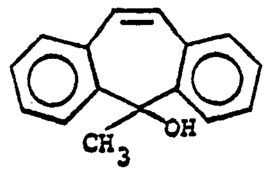

2

which carbinol of formula 2 is thereafter submitted to the reaction steps of claim 4.

6. Process for the preparation of the racemic compound of structural formula 5:

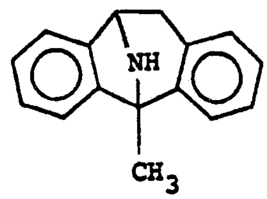

5

or salts thereof, characterized in that the compound of formula 7:

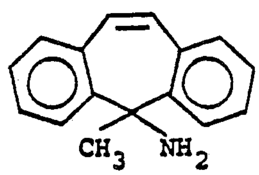

7

is reacted with a strong base in a benzenoid aromatic solvent, dimethylsulfoxide, dimethylformamide or mixtures thereof at a temperature in the range of 0° to 190°C to yield the product of formula 5.

7. Process according to patent claim 6, characterized in that the compound of formula 6:

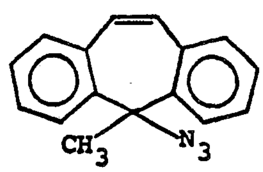

6

is reacted with hydrogen in a solvent in the presence of a noble metal catalyst to produce the compound of structural formula 7:

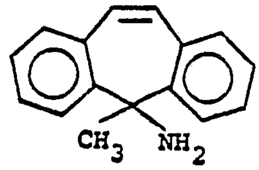

7

and that thereafter the compound of formula 7 is submitted to the process of claim 6 to yield the compounds of formula 5.

8. Process according to patent claim 7, characterized in that the compound of structural formula 2:

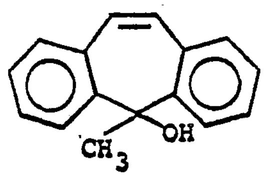

2

is reacted with sodium azide and an organic acid in a chlorinated alkane having 1—3 carbon atoms at a temperature in the range of −20° to +50°C to yield a compound of formula 6:

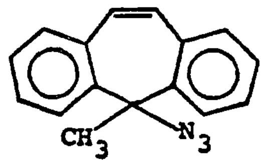

6

and that thereafter said compound of formula 6 is submitted to the reactions according to patent claim 7 to yield the final products of formula 5.

9. Process according to patent claim 8, characterized in that the compound of structural formula 1:

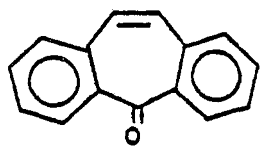

1

is reacted with a methylmagnesium halide in an ethereal solvent at a temperature in the range of −50° to +50°C to produce the carbinol of formula 2:

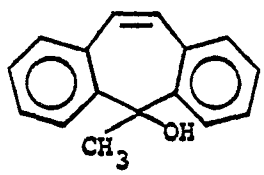

2

and that thereafter said carbinol of formula 2 is submitted to the reaction steps according to patent claim 8, to yield the final products of formula 5 or salts thereof.

**Patentansprüche**

1. Verfahren zur Herstellung der racemischen Verbindung der Strukturformel 5:

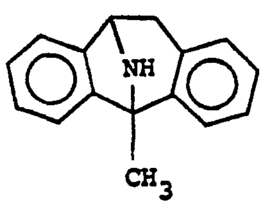

5

und Salze derselben, dadurch gekennzeichnet, dass

a) eine Verbindung der Strukturformel 3:

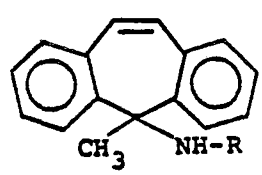

3

worin

R entweder eine Gruppe der Formel —$OR^1$ oder eine Gruppe der Formel —$NR^2R^3$ ist, wobei

$R^1$ Wasserstoff oder eine Alkylgruppe mit 1 bis 3 Kohlenstoffatomen,

$R^2$ und $R^3$ unabhängig Wasserstoff, eine Alkylgruppe mit 1 bis 3 Kohlenstoffatomen, eine Alkanoylgruppe mit 2 bis 3 Kohlenstoffatomen oder ein benzenoides Aroyl sind oder

$R^2$ und $R^3$ zusammen Tetramethylen oder Pentamethylen darstellen,

in einem benzenoiden aromatischen Lösungsmittel, Dimethylsulfoxid, Dimethylformamid oder Mischungen derselben entweder mit einer starken Base bei einer Temperatur von 0° bis 190°C behandelt wird, oder

in diesem Lösungsmittel ohne eine starke Base auf Temperaturen oberhalb etwa 100°C erhitzt wird, um die Verbindung der Strukturformel 4 zu gewinnen:

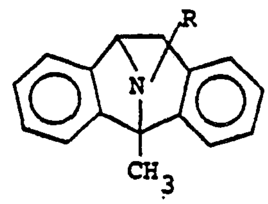

4

und dass

b) die Verbindung von Formel 4 mit Wasserstoff in einem Lösungsmittel in Gegenwart eines Edelmetallkatalysators umgesetzt wird, um die Verbindung der Formel 5 zu ergeben.

2. Verfahren nach Anspruch 1, dadurch gekennzeichnet, dass in der Verbindung der Formel 3 R —OH, —$OCH_3$, —$NH_2$ oder —$NHCOC_6H_5$ ist.

3. Verfahren nach Anspruch 1 oder 2, dadurch gekennzeichnet, dass in der Verbindung der Formel 3 R Hydroxy ist.

4. Verfahren nach einem der Ansprüche 1 bis 3, dadurch gekennzeichnet, dass die Verbindung der Formel 2:

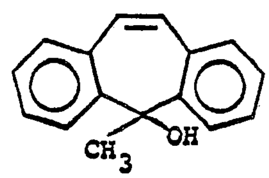

2

mit einem Amin der Formel 6 umgesetzt wird:

$$NH_2—R \qquad 6$$

worin

R entweder eine Gruppe der Formel —$OR^1$ oder eine Gruppe der Formel —$NR^2R^3$ ist, wobei

$R^1$ Wasserstoff oder eine Alkylgruppe mit 1 bis 3 Kohlenstoffatomen,

$R^2$ und $R^3$ unabhängig Wasserstoff, eine Alkylgruppe mit 1 bis 3 Kohlenstoffatomen, eine Alkanoylgruppe mit 2 bis 3 Kohlenstoffatomen oder ein benzenoides Aroyl sind, oder

$R^2$ und $R^3$ zusammen Tetramethylen oder Pentamethylen darstellen, und wobei die Reaktion in Gegenwart einer organischen Säure mittlerer Stärke in einem Chloralkan von 1 bis 3 Kohlenstoffatomen bei −20° bis +50°C durchgeführt wird, um eine substituierte Aminoverbindung der Strukturformel 3 zu ergeben:

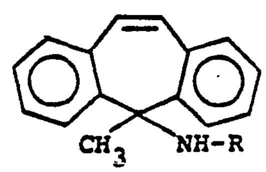

3

welche danach in die Reaktionsstufe 1 und 2 von Anspruch 1 überführt wird, um das Endprodukt der Formel 5 zu ergeben.

5. Verfahren nach Anspruch 4, dadurch gekennzeichnet, dass das Keton der Formel 1

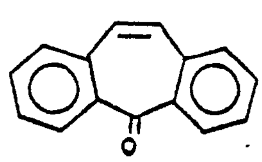

1

mit einem Methylmagnesiumhalogenid in einem etherischen Lösungsmittel bei einer Temperatur im Bereich von −50° bis +50°C umgesetzt wird, um das Carbinol der Strukturformel 2 zu ergeben:

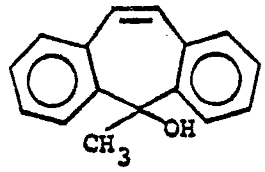

2

welchs danach den Reaktionsstufen von Anspruch 4 zugeführt wird.

6. Verfahren zur Herstellung der racemischen Verbindung der Strukturformel 5:

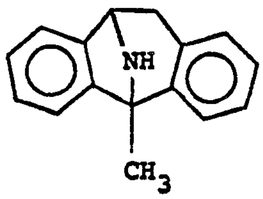

5

oder Salzen derselben, dadurch gekennzeichnet, dass die Verbindung von Formel 7:

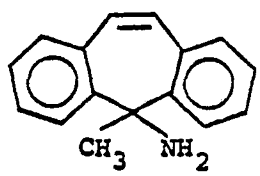

7

mit einer starken Base in einem benzenoiden aromatischen Lösungsmittel, Dimethylsulfoxid, Dimethylformamid oder Gemischen derselben bei einer Temperatur im Bereich von 0° bis 190°C umgesetzt wird, um das Produkt der Formel 5 zu ergeben.

7. Verfahren nach Anspruch 6, dadurch gekennzeichnet, dass die Verbindung von Formel 6:

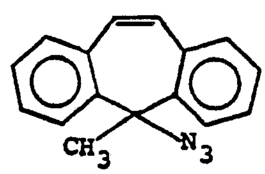

6

mit Wasserstoff in einem Lösungsmittel in Gegenwart eines Edelmetallkatalysators umgesetzt wird, um die Verbindung der Strukturformel 7 zu ergeben:

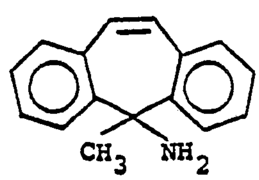

7

und dass danach die Verbinding der Formel 7 dem Verfahren von Anspruch 6 zugeführt wird, um die Verbindung von Formel 5 zu ergeben.

8. Verfahren nach Anspruch 7, dadurch gekennzeichnet, dass die Verbindung der Strukturformel 2:

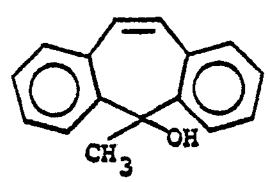

2

mit Natriumazid und einer organischen Säure in einem Chloralkan von 1 bis 3 Kohlenstoffatomen bei einer Temperatur im Bereich von −20° bis +50°C umgesetzt wird, um eine Verbindung von Formel 6 zu ergeben:

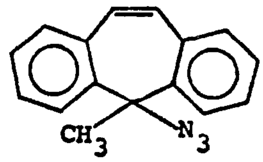

6

und dass danach die Verbindung der Formel 6 den Reaktionen gemäss Anspruch 7 zugeführt wird, um die Endprodukte der Formel 5 zu erhalten.

9. Verfahren nach Anspruch 8, dadurch gekennzeichnet, dass die Verbindung der Strukturformel 1:

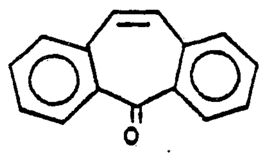

1

mit einem Methylmagnesiumhalogenid in einem etherischen Lösungsmittel bei einer Temperatur im Bereich von −50° bis +50°C umgesetzt wird, um das Carbinol der Formel 2 zu erhalten:

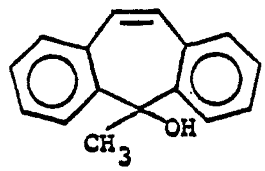

2

und dass danach dieses Carbinol der Formel 2 den Reaktionsstufen gemäss Anspruch 8 zugeführt wird, um die Endprodukte der Formel 5 oder Salze derselben zu erhalten.

**Revendications**

1. Procédé de préparation du composé racémique de formule structurale *5*:

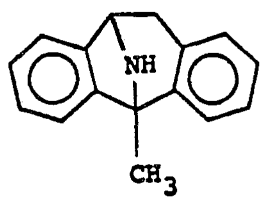

5

et de ses sels, caractérisé en ce que:

(a) on traite un composé de formule structurale *3*:

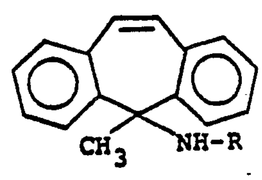

3

dans laquelle R est soit un groupe de formule —$OR^1$ soit un groupe de formule —$NR^2R^3$ dans lesquels, $R^1$ est un atome d'hydrogène ou un radical alkyle de 1 à 3 atomes de carbone, $R^2$ et $R^3$ représentent chacun indépendamment un atome d'hydrogène, un radical alkyle de 1 à 3 atomes de carbone, un radical alcanoyle de 2 à 3 atomes de carbone ou un aroyle du type benzénique ou bien $R^2$ et $R^3$ ensemble représentent un groupe tétraméthylène ou pentaméthylène, dans un solvant aromatique du type benzénique, le diméthylsulfoxyde, le diméthylformamide ou des mélanges de ceux-ci, soit avec une base forte à une température de 0 à 190°C, soit on chauffe dans ledit solvant en l'absence d'une base forte au-dessus de 100°C environ pour obtenir le composé de formule structurale 4:

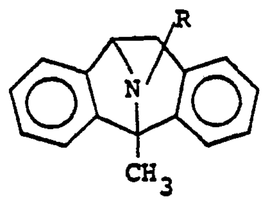

4

et èn ce que

(b) on fait réagir le composé de formule 4 avec de l'hydrogène dans un solvant en présence d'un catalyseur de métal noble pour obtenir les composés de formule 5.

2. Procédé selon la revendication 1, caractérisé en ce que dans le composé de formule 3, R est —OH, $—OCH_3$, $—NH_2$ ou $—NHCOC_6H_5$.

3. Procédé selon la revendication 1 ou 2, caractérisé en ce que dans le composé de formule 3, R est un radical hydroxy.

4. Procédé selon l'une quelconque des revendications 1 à 3, caractérisé en ce qu'on fait réagir le composé de formule 2:

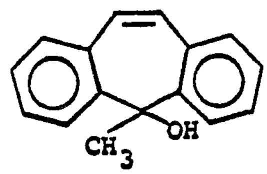

2

avec une amine de formule 6:

$$NH_2—R \qquad 6$$

dans laquelle R est ou bien un radical de formule $—OR^1$ ou bien de formule $—NR^2R^3$ dans lesquelles $R^1$ est un atome d'hydrogène ou un radical alkyle de 1 à 3 atomes de carbone, $R^2$ et $R^3$ représentent indépendamment un atome d'hydrogène, un radical alkyle de 1 à 3 atomes de carbone, un radical alcanoyle de 2 à 3 atomes de carbone ou un aroyle du type benzénique ou bien $R^2$ et $R^3$ ensemble représentent un groupe tétraméthylène ou pentaméthylène et dans lequel on effectue la réaction en présence d'un acide organique de force intermédiaire dans un alcane chloré de 1 à 3 atomes de carbone à une température de −20 à +5°C pour obtenir un composé amino substitué de formule structurale 3:

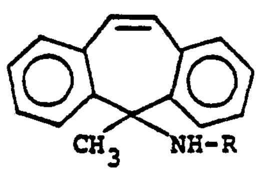

3

qu'on soumet ensuite aux étapes de réaction 1 et 2 de la revendication 1 afin d'obtenir le produit final de formule 5.

5. Procédé selon la revendication 4, caractérisé en ce qu'on fait réagir le cétone de formule 1:

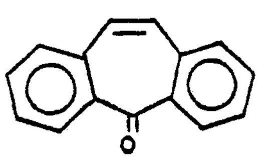

1

avec un halogènure de méthylmagnésium dans un solvant éthéré à une température de −50 à +50°C pour obtenir le carbinol de formule structurale 2:

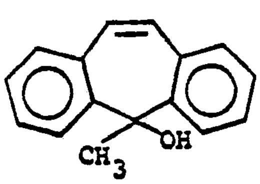

2

lequel carbinol de formule 2 est soumis ensuite aux étapes de réaction de la revendication 4.

6. Procédé de préparation du composé racémique de formule structurale 5:

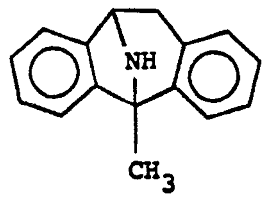

5

ou de ses sels, caractérisé en ce qu'on fait réagir le composé de formule 7:

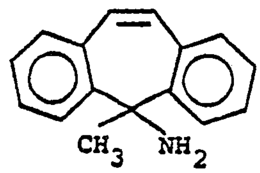

7

avec une base forte dans un solvant aromatique de type benzénique, le diméthylsulfoxyde, le diméthylformamide, ou des mélanges de ceux-ci à une température de 0 à 190°C pour obtenir le produit de formule 5.

7. Procédé selon la revendication 6, caractérisé en ce que l'on fait réagir le composé de formule 6:

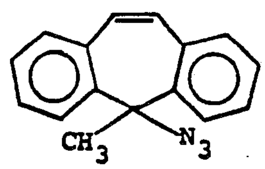

6

avec de l'hydrogène dans un solvant en présence d'un catalyseur de métal noble pour obtenir le composé de formule structurale 7:

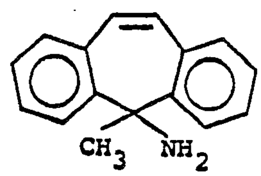

7

et en ce que par le suite on soumet le composé de formule 7 au procédé de la revendication 6 pour obtenir les composés de formule 5.

8. Procédé selon la revendication 7, caractérisé en ce que l'on fait réagir le composé de formule structurale 2:

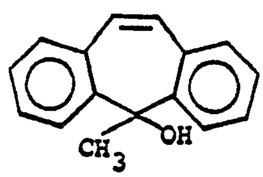

2

avec un azide de sodium et un acide organique dans un alcane chloré contenant de 1 à 3 atomes de carbone à une température de −20 à +50°C pour obtenir un composé de formule 6:

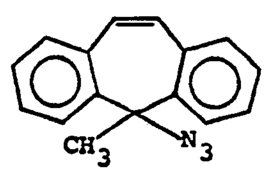

6

et en ce qu'ensuite on soumet ledit composé de formule 6 aux réactions selon la revendication 7 pour obtenir les produits finals de formule 5.

9. Procédé selon la revendication 8, caractérisé en ce que l'on fait réagir le composé de formule structurale 1:

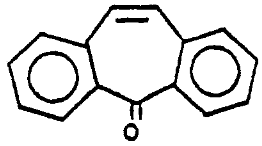

1

avec un halogénure de méthylmagnésium dans un solvant éthéré à une température de −50 à +50°C pour produire le carbinol de formule 2:

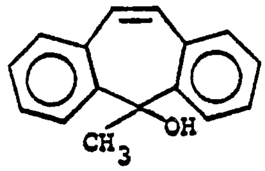

2

et en ce qu'ensuite on soumet ledit carbinol de formule 2 aux étapes de réaction selon la revendication 8 pour obtenir les produits finals de formule 5 ou leurs sels.